# EUROPÄISCHE PATENTSCHRIFT

(11) **EP 1 521 739 B1**
(45) Veröffentlichungstag und Bekanntmachung des Hinweises auf die Patenterteilung: **29.09.2010**
(21) Anmeldenummer: 03763693.3
(22) Anmeldetag: 03.07.2003
(51) Int. Cl.: C07C 273/18, C07C 275/06

(54) **VERFAHREN ZUR KONTINUIERLICHEN HERSTELLUNG VON TETRASUBSTITUIERTEN HARNSTOFFEN**
METHOD FOR THE CONTINUOUS PRODUCTION OF TETRA-SUBSTITUTED UREAS
PROCEDE DE PRODUCTION CONTINUE D'UREES TETRASUBSTITUEES

(30) Priorität: 10.07.2002 DE 10231085
(43) Veröffentlichungstag der Anmeldung: 13.04.2005
(73) Patentinhaber: BASF SE, 67056 Ludwigshafen (DE)
(72) Erfinder: HENKELMANN, Jochem, 68165 Mannheim (DE); WEBER, Theodor, 67063 Ludwigshafen (DE); ROHDE, Thorsten, 68305 Mannheim (DE); BUSCH, Ralph, 67549 Worms (DE)
(86) Internationale Anmeldenummer: PCT/EP2003/007083
(87) Internationale Veröffentlichungsnummer: WO 2004/007436

(56) Entgegenhaltungen:
- WO-A-94/22939

## Beschreibung

Die vorliegende Erfindung betrifft ein Verfahren zur Herstellung von tetrasubstituierten Harnstoffen durch Umsetzung der entsprechenden Amine mit Phosgen in Gegenwart einer wässrigen anorganischen Base bei einer Temperatur im Bereich von 0 bis 150°C.

N-substituierte Harnstoffe finden eine breite Anwendung in der Herstellung von Pflanzenschutzmitteln, Pharmazeutika und Farbstoffen. Des Weiteren werden sie als Weichmacher und Stabilisatoren in Kunststoffen, als Schmiermittel sowie als Katalysatoren, beispielsweise als Phosgenierungskatalysatoren, eingesetzt. Ferner werden N-substituierte Harnstoffe als polare aprotische Lösungsmittel eingesetzt und haben hier besondere Bedeutung als Ersatz für hochtoxische Phosphorsäureamide wie Hexamethylphosphorsäuretriamid (HMPA) oder Hexamethylphosphorigsäuretriamid (HMPT). N-Alkylharnstoffe und N-Polyalkylenharnstoffe werden zudem als Additive in der Herstellung von Aminokunststoffen verwendet.

N-substituierte Harnstoffe werden technisch überwiegend durch Transamidierung von Harnstoff mit Aminen, durch Alkylierung von Harnstoff mit Alkoholen, durch Umsetzung von Aminen mit Cyanaten sowie durch Phosgenierung von Aminen hergestellt (siehe Ullmann's Encyclopedia of Industrial Chemistry, 6th edition, 2000 electronic release, Chapter "UREA - Urea derivatives").

J5 9031-752-A und US 3,681,457 beschreiben die diskontinuierliche Herstellung von Tetramethylharnstoff durch Einleiten von Phosgen in eine Dimethylamin und wässrige Natron- oder Kalilauge enthaltende Vorlage und anschließender Extraktion der wässrigen Phase mit einem wasserunlöslichen Lösungsmittel, wie n-Hexan, Chloroform oder Ethylendichlorid, und Isolation des Tetramethylharnstoffs aus der organischen Phase.

US 5,132,423 offenbart die die diskontinuierliche Herstellung von Tetraethylharnstoff durch Einleiten von Phosgen in eine Diethylamin, Methylenchlorid und wässrige Natronlauge enthaltende Vorlage, anschließende Trennung der beiden Phasen nach erfolgter Umsetzung und Isolation des Tetraethylharnstoffs aus der organischen Phase.

EP-A 0 248 220 lehrt die diskontinuierliche Herstellung von cyclischen Harnstoffen durch Einleiten von Phosgen in eine N,N'-Dialkyldiamin und wässrige Natronlauge enthaltende Vorlage und Extraktion des erhaltenen Gemisches mit 1,2-Dichlorethan.

WO 94/22939 beschreibt ebenfalls ein Verfahren zur Herstellung von tetra-substituierten Harnstoffen.

W.A. Skinner et al. in J. Pharm. Sci. 68, 1979, Seite 391 bis 392 lehren die diskontinuierliche Herstellung von Tetrabutylharnstoff durch tropfenweise Zugabe von Dibutylamin in eine Benzol, Phosgen und Kaliumcarbonat enthaltende Vorlage, anschließendes dreistündiges Rühren bei Raumtemperatur, weitere Zugabe an Dibutylamin, dreistündiges Erhitzen unter Rückfluß, anschließendes Abfiltrieren der festen, Kaliumcarbonat und -chlorid enthaltenden Phase und destillativer Aufarbeitung des Filtrats. Laut Herstellungsvorschrift wurden insgesamt 31,25 mL Dibutylamin (0,184 mol) eingesetzt. Bei einem Gesamtvolumen der flüssigen Phase von etwa 0,28 L (Benzol, Phosgen, Kaliumcarbonat und Dibutylamin) und einer Gesamtzeit für die Umsetzung von 6,5 Stunden (0,5 Stunden tropfenweise Zugabe an Dibutylamin, 3 Stunden Rühren bei Raumtemperatur und 3 Stunden Erhitzen unter Rückfluß) ergibt sich selbst bei einer angenommenen Ausbeute an Tetrabutylharnstoff von 100% nur eine Raum-Zeit-Ausbeute von maximal 14 g/L·h.

Die oben genannten Verfahren sind insbesondere bei der Herstellung von größeren Mengen an tetrasubstituierten Harnstoffen sehr arbeits- und kostenintensiv, da in Abhängigkeit von der Reaktorgröße gegebenenfalls mehrere Ansätze hintereinander durchzuführen sind. So ist der Reaktionsapparat zu Beginn mit dem entsprechenden Amin und wässriger Lauge zu befüllen und auf Reaktionstemperatur zu bringen. Erst bei der anschließenden Einleitung von Phosgen startet dann auch die chemische Umsetzung. Nach erfolgter Umsetzung ist der Reaktionsapparat zu entleeren und für den nächsten Ansatz vorzurichten. Der Reaktionsapparat wird somit nur zu einem gewissen Zeitanteil für die eigentliche chemische Umsetzung genutzt, mit der Folge, dass auch eine niedrige Raum-Zeit-Ausbeute resultiert.

Des Weiteren ist bei den beiden oben genannten Verfahren zur Herstellung von Tetramethylharnstoff die nach der Umsetzung zu erfolgende Extraktion des Tetramethylharnstoffs mit einem organischen Lösungsmittel von Nachteil, da dies einen weiteren separaten Verfahrensschritt bedeutet.

Die Aufgabe der vorliegenden Erfindung bestand darin, ein Verfahren zur Herstellung von tetrasubstituierten Harnstoffen zu finden, welches die oben genannten Nachteile nicht besitzt und die Herstellung größerer Mengen mit einer hohe Raum-Zeit-Ausbeute ermöglicht.

Demgemäß wurde ein Verfahren zur Herstellung von tetrasubstituierten Harnstoffen durch Umsetzung der entsprechenden Amine mit Phosgen in Gegenwart einer wässrigen anorganischen Base bei einer Temperatur im Bereich von 0 bis 150°C gefunden, das dadurch gekennzeichnet ist, dass man
das entsprechende Amin, das Phosgen und die wässrige anorganische Base dem Reaktionsapparat im Mittel kontinuierlich zuführt,
durch die Auswahl der herzustellenden tetrasubstituierten Harnstoffe, durch die Mengenverhältnisse der zuzuführenden Stoffe und Stoffgemische, durch die Temperatur während der Umsetzung und gegebenenfalls durch die Zufuhr eines organischen, mit Wasser nicht vollständig mischbaren Lösungsmittels im Reaktionsapparat ein Zweiphasensystem bildet, und
das Reaktionsgemisch aus dem Reaktionsapparat im Mittel kontinuierlich abführt.

Das erfindungsgemäße Verfahren ist somit ein kontinuierlich arbeitendes Verfahren und weist im Reaktionsapparat ein Zweiphasensystem auf. Dieses umfasst eine wässrige, flüssige Phase und eine organische, flüssige Phase.

Beim erfindungsgemäßen Verfahren sind das entsprechende Amin, das Phosgen und die wässrige anorganische Base dem Reaktionsapparat im Mittel kontinuierlich zuzuführen und das Reaktionsgemisch aus dem Reaktionsapparat im Mittel kontinuierlich abzuführen. Unter einer im Mittel kontinuierlichen Zufuhr oder Abfuhr seien auch periodische oder aperiodische Schwankungen in der zu- oder abgeführten Menge bis hin zur pulsartigen Zu- oder Abfuhr zu verstehen. Bevorzugt ist die Zufuhr oder Abfuhr einer konstanten oder nahezu konstanten Menge.

Um auch über einen längeren Zeitraum hinweg einen kontinuierlichen und konstanten Betrieb des erfindungsgemäßen Verfahrens zu ermöglichen, ist es vorteilhaft, den Reaktionsapparat bei konstantem oder nahezu konstantem Füllvolumen (Füllstand) zu betreiben und die abzuführende Menge über das gewünschte Füllvolumen (Füllstand) zu regeln.

Die Bildung eines Zweiphasensystems im Reaktionsapparat wird beim erfindungsgemäßen Verfahren durch verschiedene Maßnahmen erreicht:
a) Durch die Auswahl der herzustellenden tetrasubstituierten Harnstoffe
   Je nach der Art der Substituenten sind die herzustellenden tetrasubstituierten Harnstoffe in Wasser vollständig löslich, im gewissen Maße löslich oder nur sehr gering oder nahezu unlöslich. Bei tetrasubstituierten Harnstoffen, welche nicht vollständig in Wasser löslich sind, kann sich daher bereits, abhängig von den relativen Mengenverhältnissen und der Temperatur, eine organische Phase ausbilden.
b) Durch die Mengenverhältnisse der zuzuführenden Stoffe und Stoffgemische
   Die Mengenverhältnisse der zuzuführenden Stoffe und Stoffgemische haben einen Einfluß auf die im Reaktionsapparat vorliegenden Mengenverhältnisse der anorganischen und organischen Stoffe und somit in Verbindung mit den Löslichkeitseigenschaften der tetrasubstituierten Harnstoffe auch auf die Ausbildung eines Zweiphasensystems. So ist es beispielsweise vorteilhaft, bei der Herstellung von tetrasubstituierten Harnstoffen, welche im Wasser im gewissen Maße löslich sind, tendenziell eine geringere Menge an wässriger Phase einzusetzen. Um hierbei dennoch die erforderliche Menge an anorganischer Base bereitzustellen, ist gegebenenfalls eine höher konzentrierte wässrige anorganische Base zuzuführen.
c) Durch die Temperatur während der Umsetzung
   Da die Löslichkeitseigenschaften der tetrasubstituierten Harnstoffe auch von der Temperatur abhängig sind, hat die vorliegende Temperatur auch einen Einfluß auf die Ausbildung eines Zweiphasensystems. Es sei jedoch betont, dass bei der Wahl der Reaktionstemperatur auch andere Größen, wie beispielsweise die Reaktionsgeschwindigkeit, der im Reaktionsapparat vorliegende Druck oder die Bildung unerwünschter Nebenprodukte, beeinflusst werden und vorteilhafterweise gegeneinander abzuwägen sind.
d) Gegebenenfalls durch die Zufuhr eines organischen, mit Wasser nicht vollständig mischbaren Lösungsmittels im Reaktionsapparat
   Des Weiteren kann beim erfindungsgemäßen Verfahren auch ein organisches, mit Wasser nicht vollständig mischbares Lösungsmittel zugeführt werden. Dieses ermöglicht auch die Ausbildung eines Zweiphasensystems, bei denen die oben aufgeführten Maßnahmen a) bis c) nicht ausreichend sind. So vermag beispielsweise die Zufuhr eines organischen, mit Wasser nicht vollständig mischbaren Lösungsmittels auch bei der Herstellung von vollständig mit Wasser mischbaren tetrasubstituierten Harnstoffen eine organische Phase auszubilden. Aufgrund der Löslichkeitseigenschaft des tetrasubstituierten Harnstoffs liegt dieser in der Regel dann zu einem beträchtlichen Teil in der organischen Phase vor. Des Weiteren vermag der Zusatz eines organischen, mit Wasser nicht vollständig mischbaren Lösungsmittels tetrasubstituierte Harnstoffe, welche unter den Reaktionsbedingungen als Feststoff vorliegen würden, in Lösung zu halten.

Die gegebenenfalls einzusetzenden organischen, mit Wasser nicht vollständig mischbaren Lösungsmittel sollten vorteilhafterweise unter den eingestellten Reaktionsbedingungen chemisch inert sein, das heißt chemisch nicht mit den eingesetzten Verbindungen reagieren, eine gute Lösungseigenschaft gegenüber den zu lösenden tetrasubstituierten Harnstoff aufweisen und auf einfache Art und Weise, beispielsweise destillativ, vom tetrasubstituierten Harnstoff abtrennbar sein. Als geeignete Lösungsmittel seien chlorierte Kohlenwasserstoffe, wie beispielsweise Dichlormethan, Trichlormethan und Dichlorethan; gesättigte aliphatische oder cyclische Kohlenwasserstoffe, wie beispielsweise Hexan, Heptan, isoOctan und Cyclohexan; aromatische Kohlenwasserstoffe, wie beispielsweise Benzol, Toluol und Xylol; sowie halogenierte aromatische Kohlenwasserstoffe, wie beispielsweise Chlorbenzol und Dichlorbenzol genannt.

Für jedes System kann durch einfache Versuche ermittelt werden, ob unter den gewünschten Bedingungen ein Zweiphasensystem vorliegt oder nicht. Falls kein Zweiphasensystem vorliegt, kann bei einem vorgegebenen tetrasubstituierten Harnstoff durch die oben genannten Maßnahmen b), c) und/oder d) ein Zweiphasensystem gezielt eingestellt werden.

Durch die oben genannten Maßnahmen wird die Bildung eines Zweiphasensystems sichergestellt. Prinzipiell ist es für das erfindungsgemäße Verfahren umso vorteilhafter, je geringer die Menge des in der wässrigen Phase gelösten tetrasubstituierten Harnstoffs ist. Bevorzugt liegt beim erfindungsgemäßen Verfahren der in der wässrigen Phase vorhandene Anteil an tetrasubstituiertem Harnstoff bei ≤5% und besonders bevorzugt bei ≤1% der Gesamtmenge an vorhandenem tetrasubstituiertem Harnstoff.

Die durch das erfindungsgemäße Verfahren herstellbaren tetrasubstituierten Harnstoffe besitzen die allgemeine Formel (I) 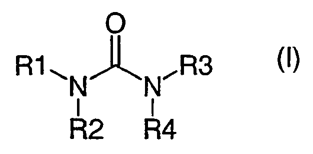 in der die Reste R¹ bis R⁴ für Kohlenstoff enthaltende organische Reste stehen, wobei diese gegebenenfalls auch miteinander verbunden sein können.

Unter einem Kohlenstoff enthaltenden organischen Rest ist ein unsubstituierter oder substituierter, aliphatischer, aromatischer oder araliphatischer Rest zu verstehen. Dieser kann ein oder mehrere Heteroatome, wie etwa Sauerstoff, Stickstoff, Schwefel oder Phosphor enthalten, beispielsweise -O-, -S-, -NR-, -CO-, -N=, -PR- und/oder -PR₂ und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe (bei dem Rest R handelt es sich hierbei ebenfalls um einen Kohlenstoff enthaltenden organischen Rest). Bei dem Kohlenstoff enthaltenden organischen Rest kann es sich um einen einwertigen oder auch um einen zwei-, drei- oder vierwertigen Rest handeln.

Sind zwei oder mehrere der Reste R¹ bis R⁴ miteinander verbunden, so sind dies bevorzugt die Reste R² mit R⁴ oder R¹ mit R² und/oder R³ mit R⁴.

Als einwertige Reste stehen die Reste R¹ bis R⁴ unabhängig voneinander bevorzugt für
* einen unverzweigten oder verzweigten, acyclischen oder cyclischen, unsubstituierten oder substituierten Alkylrest mit 1 bis 30 aliphatischen Kohlenstoffatomen, bei dem eine oder mehrere der CH₂-Gruppen auch durch Heteroatome, wie -O- oder -S-, oder durch Heteroatom enthaltende Gruppen, wie -CO- oder -NR-, ersetzt sein können und bei dem ein oder mehrere der Wasserstoffatome durch Substituenten, wie beispielsweise Arylgruppen oder funktionelle Gruppen ersetzt sein können; oder für
* einen unsubstituierten oder substituierten aromatischen Rest mit 3 bis 30 Kohlenstoffatomen und einem Ring oder zwei oder drei kondensierten Ringen, bei dem eines oder mehrere Ringatome durch Heteroatome, wie beispielsweise Stickstoff, substituiert sein können und bei dem ein oder mehrere der Wasserstoffatome durch Substituenten, wie beispielsweise Alkyl-, Arylgruppen oder funktionelle Gruppen ersetzt sein können.

Als zweiwertige Reste stehen bevorzugt die Reste R¹ mit R² und/ oder R³ mit R⁴ bevorzugt für
* einen unverzweigten oder verzweigten, acyclischen oder cyclischen, unsubstituierten oder substituierten C₄- bis C₂₀-Alkylenrest ("zweiwertiger Alkylrest") mit 4 bis 10 Atomen in der Alkylenkette, bei dem CH₂-Gruppen auch durch Heterogruppen, wie beispielsweise -CO-, -O- oder -NR- ersetzt sein können und bei dem ein oder mehrere der Wasserstoffatome durch Substituenten, wie beispielsweise Alkyl- oder Arylgruppen ersetzt sein können.

Als zweiwertige Reste stehen bevorzugt die Reste R² mit R⁴ bevorzugt für
* einen unverzweigten oder verzweigten, acyclischen oder cyclischen, unsubstituierten oder substituierten C₂- bis C₂₀-Alkylenrest ("zweiwertiger Alkylrest") mit 2 bis 10 Atomen in der Alkylenkette, bei dem CH₂-Gruppen auch durch Heterogruppen, wie beispielsweise -CO-, -O- oder -NR- ersetzt sein können und bei dem ein oder mehrere der Wasserstoffatome durch Substituenten, wie beispielsweise Alkyl- oder Arylgruppen ersetzt sein können.

Besonders bevorzugt werden tetrasubstituierte Harnstoffe (I) hergestellt,
(i) deren Reste R¹ bis R⁴
   * einen unverzweigten oder verzweigten C₁- bis C₂₀-Alkylrest, wie beispielsweise Methyl, Ethyl, 1-Propyl, 2-Propyl (sek.-Propyl), 1-Butyl, 2-Butyl (sek.-Butyl), 2-Methyl-1-propyl (iso-Butyl), 2-Methyl-2-propyl (tert.-Butyl), 1-Pentyl, 2-Pentyl, 3-Pentyl, 2-Methyl-2-butyl (tert.-Amyl), 1-Hexyl, 2-Hexyl, 3-Hexyl, 2-Methyl-2-pentyl oder 3-Methyl-3-pentyl;
   * einen unverzweigten oder verzweigten C₅- bis C₂₀-Cycloalkylrest, wie beispielsweise Cyclopentyl, Cyclohexyl oder Cyclooctyl; oder
   * einen unsubstituierten oder mit einem oder mehreren C₁-bis C₄-Alkylresten substituierten C₆- bis C₂₀-Aryl- oder C₃- bis C₂₀-Heteroaryl-Rest, wie beispielsweise Phenyl, 2-Methylphenyl (o-Tolyl), 3-Methylphenyl (m-Tolyl), 4-Methylphenyl (p-Tolyl), 2,6-Dimethylphenyl, 2,4-Dimethylphenyl, 2,4,6-Trimethylphenyl, 2-Methoxyphenyl, 3-Methoxyphenyl, 4-Methoxyphenyl, 2-Pyridyl, 3-Pyridyl, 4-Pyridyl, 3-Pyridazinyl, 4-Pyridazinyl, 5-Pyridazinyl, 2-Pyrimidinyl, 4-Pyrimidinyl, 5-Pyrimidinyl, 2-Pyrazinyl, 2-(1,3,5-Triazin)yl, 1-Naphthyl, 2-Naphthyl, 2-Chinolyl, 8-Chinolyl, 1-Isochinolyl oder 8-Isochinolyl bedeuten;
(ii) deren Reste R¹ mit R² und R³ mit R⁴ verbunden sind und
   * einen unverzweigten oder verzweigten, unsubstituierten oder substituierten C₄- bis C₁₀-Alkylenrest ("zweiwertiger Alkylrest") mit 4 bis 10 Atomen in der Alkylenkette, bei dem CH₂-Gruppen auch durch Heterogruppen, wie beispielsweise -CO-, -O- oder -NR- ersetzt sein können und bei dem ein oder mehrere der Wasserstoffatome durch Substituenten, wie beispielsweise Alkyl- oder Arylgruppen ersetzt sein können, wie beispielsweise Butylen, Pentylen, Hexylen, -CH₂CH₂-O-CH₂CH₂- oder - CH₂CH₂-NR-CH₂CH₂- bedeuten; oder
(iii) deren Reste R¹ und R⁴ wie unter (i) genannt und die Reste R² mit R³ verbunden sind und
   * einen unverzweigten oder verzweigten, unsubstituierten oder substituierten C₂- bis C₁₀-Alkylenrest ("zweiwertiger Alkylrest") mit 2 bis 10 Atomen in der Alkylenkette, bei dem CH₂-Gruppen auch durch Heterogruppen, wie beispielsweise -CO-, -O- oder -NR- ersetzt sein können und bei dem ein oder mehrere der Wasserstoffatome durch Substituenten, wie beispielsweise Alkyl- oder Arylgruppen ersetzt sein können, wie beispielsweise Ethylen, Propylen, Butylen, Pentylen, Hexylen, -CH₂CH₂-O-CH₂CH₂- oder -CH₂CH₂-NR-CH₂CH₂- bedeuten.

Ganz besonders bevorzugt werden beim erfindungsgemäßen Verfahren als tetrasubstituierte Harnstoffe die symmetrisch tetrasubstituierten Harnstoffe N,N,N',N'-Tetramethylharnstoff, N,N,N',N'-Tetraethylharnstoff, N,N,N',N'-Tetrapropylharnstoff, N,N,N',N'-Tetrabutylharnstoff, N,N,N',N'-Tetrapentylharnstoff, N,N,N',N'-Tetrahexylharnstoff, N,N,N',N'-Tetra(cyclopropyl)-harnstoff, N,N,N',N'-Tetra(cyclohexyl)-harnstoff, N,N,N',N'-Tetraphenylharnstoff, Bis(butylen)-harnstoff, Bis(pentylen)harnstoff, N,N'-Dimethylethylenharnstoff, N,N'-Dimethylpropylenharnstoff, N,N'-Dimethyl-(2-(methylaza)-propylen)-harnstoff und N,N'-Dimethyl-(3-(methylaza)-pentylen)-harnstoff hergestellt.

Insbesondere werden beim erfindungsgemäßen Verfahren als tetrasubstituierte Harnstoffe N,N,N',N'-Tetrabutylharnstoff, N,N'-Dimethylethylenharnstoff und N,N'-Dimethylpropylenharnstoff hergestellt.

Als entsprechende Amine sind die Amine HNR¹R² sowie HNR³R⁴ einzusetzen, wobei entsprechend der obigen Beschreibung die Reste R¹ bis R⁴ gegebenenfalls auch miteinander verbunden sein können. So ist beispielsweise bei der Herstellung von N,N,N',N'-Tetrabutylharnstoff als entsprechendes Amin Dibutylamin und bei der Herstellung von N,N'-Dimethylpropylenharnstoff N,N'-Dimethylpropan-1,3-diamin einzusetzen.

Das zuzuführende Amin kann auf verschiedene Art und Weise erfolgen. Es kann prinzipiell flüssig und/oder gasförmig zugegeben werden. Des Weiteren kann das zuzuführende Amin beispielsweise pur oder verdünnt mit einem Inertgas oder einem organischen, mit Wasser nicht vollständig mischbaren Lösungsmittel zugegeben werden.

Die Zufuhr des Phosgens kann ebenfalls auf verschiedene Art und Weise, beispielsweise flüssig und/oder gasförmig beziehungsweise pur oder mit einem Inertgas oder einem organischen, mit Wasser nicht vollständig mischbaren Lösungsmittel verdünnt, erfolgen. Bevorzugt ist die Zugabe von flüssigem oder gasförmigen Phosgen ohne Verdünnung.

Bei der wässrigen anorganischen Base handelt es sich um eine wässrige Lösung einer anorganischen Base. Als Beispiele seien Ammoniakwasser, Natronlauge und Kalilauge genannt.

Die zuzuführende anorganische Base dient bei der Umsetzung der entsprechenden Amine mit Phosgen zur Bindung des formell entstehenden Chlorwasserstoffs. Um eine Konkurrenzreaktion mit dem entsprechenden Amin, welches als Edukt für die Herstellung des substituierten Harnstoffs fungiert, zu vermeiden beziehungsweise auf ein Minimum zu verringern, ist es besonders vorteilhaft, eine wässrige anorganische Base einzusetzen, welche einen niedrigeren pK_{b}-Wert, gemessen bei 25°C in wässriger Lösung, aufweist als das entsprechende Amin.

Ganz besonders bevorzugt setzt man beim erfindungsgemäßen Verfahren als wässrige anorganische Base Natronlauge und/oder Kalilauge ein.

Das molare Verhältnis zwischen dem zuzuführenden Phosgen und dem zuzuführenden entsprechenden Amin beträgt im Allgemeinen 0,3 bis 0,8 und bevorzugt 0,4 bis 0,6 bei Aminen mit einer NH-Gruppe und 0,6 bis 1,6 und bevorzugt 0,8 bis 1,2 bei Aminen mit zwei NH-Gruppen (für die Herstellung cyclischer Harnstoffe).

Das molare Verhältnis zwischen der zuzuführenden anorganischen Base und dem zuzuführenden entsprechenden Amin beträgt im Allgemeinen 0,5 bis 2 und bevorzugt 0,7 bis 1,5 bei Aminen mit einer NH-Gruppe und 1,0 bis 4 und bevorzugt 1,4 bis 3 bei Aminen mit zwei NH-Gruppen (für die Herstellung cyclischer Harnstoffe).

Bevorzugt führt man die Umsetzung bei einem pH-Wert von ≥9, besonders bevorzugt von ≥10 und ganz besonders bevorzugt von 10 bis 12 durch. Es hat sich dabei als besonders vorteilhaft erwiesen, die Zufuhr an wässriger anorganischer Base über den im Reaktionsgemisch gewünschten pH-Wert zu regeln.

Wie zuvor schon erwähnt, ist bei der Herstellung von mit Wasser vollständig oder sehr gut löslichen tetrasubstituierten Harnstoffen im Allgemeinen die Gegenwart eines organischen, mit Wasser nicht vollständig mischbaren Lösungsmittels erforderlich, um die Bildung eines Zweiphasensystems im Reaktionsapparat zu gewährleisten. Das zugeführte Lösungsmittel ist dann bei der anschließenden Aufarbeitung in der Regel wieder zu entfernen, was einen zusätzlichen Aufwand bedeutet. Tetrasubstituierte Harnstoffe mit einer geringeren Löslichkeit in Wasser sind daher durch das erfindungsgemäße Verfahren besonders vorteilhaft herstellbar, da die Menge an zuzuführendem organischen, mit Wasser nicht vollständig mischbaren Lösungsmittel wesentlich geringer sein kann beziehungsweise sogar gänzlich unterbleiben kann. Beim erfindungsgemäßen Verfahren werden daher bevorzugt tetrasubsti- - tuierten Harnstoffe hergestellt, welche eine Löslichkeit in Wasser von ≤10 g/L Wasser und besonders bevorzugt von ≤ 2 g/L Wasser, gemessen bei 25°C und Normaldruck, aufweisen.

Wie ebenfalls zuvor schon erwähnt, bedürfen tetrasubstituierte Harnstoffe, deren Schmelzpunkt oberhalb der Reaktionstemperatur liegt in der Regel ebenfalls einer Lösung in einem organischen, mit Wasser nicht vollständig mischbaren Lösungsmittel. Tetrasubstituierte Harnstoffe mit einem Schmelzpunkt unterhalb der Reaktionstemperatur sind daher durch das erfindungsgemäße Verfahren besonders vorteilhaft herstellbar, da die Menge an zuzuführendem organischen, mit Wasser nicht vollständig mischbaren Lösungsmittel wesentlich geringer sein kann beziehungsweise sogar gänzlich unterbleiben kann. Beim erfindungsgemäßen Verfahren werden daher bevorzugt tetrasubstituierten Harnstoffe hergestellt, welche einen Schmelzpunkt von ≤150°C und besonders bevorzugt von ≤ 100°C aufweisen.

Das erfindungsgemäße Verfahren führt man bevorzugt bei einer Temperatur von 10 bis 100°C und besonders bevorzugt von 50 bis 85°C durch. Bei Durchführung des erfindungsgemäßen Verfahrens beträgt der Druck im Allgemeinen 0,05 bis 1,0 MPa abs, bevorzugt 0,08 bis 0,2 MPa abs und ganz besonders bevorzugt 0,08 bis 0,12 MPa abs.

Als Reaktionsapparate können beim erfindungsgemäßen Verfahren prinzipiell die in der Fachliteratur beschriebenen Apparate für gas-flüssig-Umsetzungen, beziehungsweise im Falle des Einsatzes von flüssigem Phosgen die bekannten Apparate für flüssig-flüssig-Umsetzungen eingesetzt werden. Als geeignete Reaktionsapparate seien beispielsweise Rührkessel, Strömungsrohre (bevorzugt mit Einbauten), Blasensäulen und Schlaufenreaktoren genannt.

Bevorzugt führt man die Umsetzung in einem Rührkessel durch. Um einen effizienten Eintrag sicherzustellen, wird das Phosgen bevorzugt durch den Rührer und/oder durch Düsen eingebracht.

In einer besonders bevorzugten Variante führt man die Umsetzung in einer Kaskade von mindestens zwei Rührkesseln durch. Der erste Rührkessel fungiert dabei als sogenannter Hauptreaktor, dem unter intensiver Durchmischung die Einsatzstoffe, das heißt das entsprechende Amin, das Phosgen, die wässrige anorganische Base und gegebenenfalls das organische, mit Wasser nicht vollständig mischbare Lösungsmittel, im Mittel kontinuierlich zugeführt werden und in dem eine teilweise Umsetzung zum tetrasubstituierten Harnstoff erfolgt. Bevorzugt werden im Hauptreaktor 10 bis 95% des tetrasubstituierten Harnstoffs gebildet. Das zweiphasige Reaktionsgemisch des Hauptreaktors, welches die anorganische Base, das gebildete Chlorid, das nicht-umgesetztes Amin, das disubstituierte Carbaminsäurechlorid als Zwischenprodukt, den tetrasubstituierten Harnstoff und gegebenenfalls nicht-umgesetztes Phosgen und zugegebenes Lösungsmittel enthält, wird nun im Mittel kontinuierlich dem zweiten Rührkessel zugeführt. Dieser fungiert als sogenannter Nachreaktor, in dem die restliche Umsetzung, insbesondere die Umsetzung zwischen dem disubstituierte Carbaminsäurechlorid und dem noch freien Amin erfolgt. Im Allgemeinen werden dem Nachreaktor keine weiteren Edukte zugeführt. Bei Bedarf ist es jedoch möglich, beispielsweise zur Erreichung der Gesamtstöchiometrie, weiteres Edukt, beispielsweise Phosgen oder das entsprechende Amin zuzuführen. Auch im Nachreaktor ist eine intensive Durchmischung sicherzustellen.

In der Regel ist ein Nachreaktor ausreichend, das heißt die besonders bevorzugte Rührkesselkaskade umfasst somit zwei Rührkessel. Das Volumenverhältnis von Hauptreaktor zu Nachreaktor beträgt vorteilhafterweise 0,1 bis 4 und bevorzugt 0,5 bis 2.

Beim Einsatz eines Rührkessels erfolgt die im Mittel kontinuierliche Abfuhr des Reaktionsgemisches über eine entsprechend positionierte Abfluß- beziehungsweise Entnahmevorrichtung. Bei dieser kann es sich beispielsweise um eine Öffnung in der Wandung des Rührkessels oder um ein Tauchrohr handeln.

In einer besonders bevorzugten Variante des erfindungsgemäßen Verfahrens führt man einen Teil des Reaktionsgemisches aus dem flüssigkeitsoberflächennahen Bereich und einen weiteren Teil aus dem bodennahen Bereich des Rührkessels ab. Im Allgemeinen werden die beide abgeführten Ströme vereinigt und in die weitere Aufarbeitung oder bei einer Reaktorkaskade in den nachfolgenden Reaktor geleitet. Durch die genannte besonders bevorzugte Ausführungsform wird der Anteil an organischer Phase im Rührkessel erhöht und der Anteil an wässriger Phase herabgesetzt. Grund hierfür ist die Erkenntnis, dass trotz intensiver Durchmischung die Verteilung von organischer und wässriger Phase im Rührkessel nicht exakt gleichmäßig ist und im bodennahen Bereich ein etwas erhöhter Anteil an wässriger Phase vorliegt. Durch die genannte Erhöhung des Anteils der organischen Phase wird eine weitere Erhöhung der Raum-Zeit-Ausbeute erreicht.

Das Anfahren des kontinuierlichen Verfahrens kann auf verschiedene Art und Weise erfolgen. Es ist jedoch darauf zu achten, dass die durch die exotherme Reaktion freigesetzte Wärme entsprechend abgeführt werden kann. Daher sollte insbesondere die Zufuhr der jeweils dritten Reaktionskomponente besonders vorsichtig erfolgen. Nach dem Anspringen der Reaktion können dann üblicherweise alle drei Reaktionskomponenten kontinuierlich zugeführt werden. In einer Variante legt man das entsprechende Amin vor, leitet Phosgen ein und anschließend kontinuierlich weiteres Amin und die wässrige anorganische Base. Nach Erreichen des gewünschten Füllstands wird das Reaktionsgemisch im Mittel kontinuierlich aus dem Reaktionsapparat abgeführt. In einer anderen Variante kann man das vorgelegte Amin auch mit dem organischen, mit Wasser nicht vollständig mischbaren Lösungsmittel oder Reaktionsgemisch aus einer vorherigen Produktionskampagne verdünnen und anschließend wie zuvor beschrieben verfahren. Dadurch wird ein besonders schonendes Anfahren ermöglicht.

Unabhängig von der Art des eingesetzten Reaktionsapparats beziehungsweise der Anzahl der Reaktionsapparate wird durch das erfindungsgemäße Verfahren zunächst ein zweiphasiges Reaktionsgemisch erhalten. Die wässrige Phase enthält dabei überwiegend die gegebenenfalls überschüssige anorganische Base, das gebildete Chlorid sowie gegebenenfalls entsprechend der Löslichkeit Anteile organischer Verbindungen. Die organische Phase enthält überwiegend den tetrasubstituierten Harnstoff sowie gegebenenfalls nicht-umgesetztes Amin, nicht-umgesetztes disubstituiertes Carbaminsäurechlorid als Zwischenprodukt sowie entsprechend der Löslichkeit Anteile der wässrigen Phase. Bei der Aufarbeitung wird das zweiphasige Reaktionsgemisch üblicherweise in beide Phasen getrennt. Die kann beispielsweise in einem sogenannten Settler als Phasentrenngefäß erfolgen. Im Allgemeinen ist es vorteilhaft, die isolierte organische Phase anschließend mit Wasser zu waschen, um weitere wasserlösliche Stoffe zu entfernen. Der tetrasubstuierte Harnstoff kann nun aus der organischen Phase isoliert werden. Bevorzugt erfolgt die Isolation destillativ. In einer ersten Destillationsstufe werden dabei über Kopf die Leichtsieder, wie beispielsweise restliches Wasser sowie nicht-umgesetztes Amin abgetrennt. In einer zweiten Destillationsstufe wird dann der tetrasubstituierte Harnstoff über Kopf gewonnen.

In einer besonders bevorzugten Ausführungsform legt man im ersten Rührkessel, welcher als Hauptreaktor fungiert, das entsprechende Amin und die wässrige anorganische Base vor, erwärmt auf die gewünschte Reaktionstemperatur und gast unter Rühren Phosgen bis zum gewünschten pH-Wert ein. Aus Sicherheitsgründen ist es im Allgemeinen vorteilhaft, den Rührkessel nur zu einem Teil von etwa 20 bis 50% zu füllen. Anschließend beginnt man in der Regel mit der kontinuierlichen Zufuhr des Amins und der wässrigen anorganischen Base, wobei weiteres Phosgen entsprechend kontinuierlich eingeleitet wird. Nach Erreichen des gewünschten Füllstandes wird aus dem Hauptreaktor Reaktionsgemisch kontinuierlich in den zweiten Rührkessel, welcher als Nachreaktor fungiert, geleitet. Dieser kann untemperiert oder auch temperiert betrieben werden. Nachdem unter Rühren auch der Nachreaktor bis zum gewünschten Füllstand aufgefüllt wurde, wird auch aus diesem das Reaktionsgemisch kontinuierlich abgeführt. Das Reaktionsgemisch wird in einen sogenannten Settler zur Phasentrennung geleitet und die organische Phase mit Wasser gewaschen. Anschließend erfolgt die Aufarbeitung der organischen Phase nach den üblichen Methoden, wobei eine destillative Aufarbeitung bevorzugt ist.

Das erfindungsgemäße Verfahren ermöglicht die kontinuierliche Herstellung von tetrasubstituierten Harnstoffen unter Vermeidung der bei den technisch üblichen diskontinuierlichen Verfahren erforderlichen zeit-, arbeits- und energie-aufwendigen Arbeitsschritten mit höherer Selektivität, höherer Raum/Zeit-Ausbeute und daher höherer Produktivität in einfacher Art und Weise. So ermöglicht das erfindungsgemäße Verfahren bei der Herstellung von N,N,N',N'-Tetrabutylharnstoff eine Raum-Zeit-Ausbeute von mehreren hundert g/L·h.

Das besonders bevorzugte erfindungsgemäße Verfahren zur Herstellung von tetrasubstituierten Harnstoffen, welche eine Löslichkeit in Wasser von ≤10 g/L Wasser, gemessen bei 25°C und Normaldruck, und einen Schmelzpunkt unterhalb der Reaktionstemperatur aufweisen, ermöglicht die Umsetzung ohne Zusatz eines Lösungsmittels. Dadurch ist aufgrund der möglichen höheren Konzentration der Edukte eine besonders hohe Raum-Zeit-Ausbeute und eine besonders einfache Aufarbeitung möglich.

### Beispiele

### Beispiel 1

40 g 23,8 Gew.-%ige wässrige Natronlauge und 24 g Dibutylamin wurden in einem 0,8 L-Rührkessel mit Überlauf vorgelegt und auf 75°C erwärmt. Zum Anfahren des Reaktors wurde nun bei dieser Temperatur Phosgen eingegast, bis der pH-Wert des Reaktionsgemisches auf einen Wert im Bereich von 10 bis 11 gesunken war. Anschließend wurde bei dieser Temperatur weitere 23,8 Gew.-%ige wässrige Natronlauge und Dibutylamin zugeführt, wobei die Zufuhrmengen derart eingestellt wurden, dass im Reaktor ein pH-Wert von etwa 11 gehalten werden konnte. Nachdem der gewünschte Füllstand des Reaktors erreicht war, wurde mit der kontinuierlichen Fahrweise begonnen und bei einer Reaktionstemperatur von 75°C innerhalb von 6 Stunden 194 g (1,5 Mol) Dibutylamin, 97 g Phosgen und 518 g 23,8 Gew.-%ige Natronlauge zugefahren und das Reaktionsgemisch über den Überlauf kontinuierlich abgeführt. Der zweiphasige Austrag wurde über einen Phasenscheider in die wässrige und die organische Phase getrennt. Es wurden dabei insgesamt 231 g organische Phase erhalten. Diese enthielt 88,2 GC-Flächen-% N,N,N',N'-Tetrabutylharnstoff, 9,3 GC-Flächen-% nicht-umgesetztes Dibutylamin und 0,8 GC-Flächen-% N,N-Dibutylcarbaminsäurechlorid. Die Ausbeute an N,N,N',N'-Tetrabutylharnstoff betrug 88%, bezogen auf das eingesetzte Dibutylamin. Die während des kontinuierlichen Betriebs erreichte Raum-Zeit-Ausbeute betrug somit [(1,5 Mol)/2)-0,88·284 g/Mol] / 0,8 L·6 h = 39,1 g/L·h.

### Beispiel 2

Die Versuchsanlage umfasste eine Rührkesselkaskade mit einem 0,3 L-Rührkessel als Hauptreaktor, einem 0,5 L-Rührkessel als Nachreaktor und einem nachgeschalteten 0,2 L-Settler. Zum Anfahren der Versuchsanlage wurde der Hauptreaktor mit Rohaustrag, welcher gemäß Beispiel 1 gewonnen wurde und die organische und die wässrige Phase umfasste, gefüllt und auf 50°C erwärmt. Nun wurde mit der kontinuierlichen Fahrweise begonnen und bei einer Reaktionstemperatur von 50°C innerhalb von 5,75 Stunden 360 g (2,79 Mol) Dibutylamin, 132 g Phosgen und 1015 g 15 Gew.-%ige wässrige Natronlauge zugefahren, wobei der pH-Wert im Hauptreaktor über die Dosierung der Natronlauge auf einen Wert von etwa 11 gehalten wurde. Der Nachreaktor blieb unbeheizt. Das Reaktionsgemisch wurde kontinuierlich über einen Überlauf vom Hauptreaktor in den Nachreaktor geleitet und nach dessen Füllung über einen weiteren Überlauf in den Settler gepumpt. Dort erfolgte die Phasentrennung. Die gesammelte organische Phase des Austrags enthielt 88,2 GC-Flächen-% N,N,N',N'-Tetrabutylharnstoff, 11,3 GC-Flächen-% nicht-umgesetztes Dibutylamin und 0,15 GC-Flächen-% N,N-Dibutylcarbaminsäurechlorid.

### Beispiel 3

Die Versuchsanlage umfasste eine Rührkesselkaskade mit einem 0,5 L-Rührkessel als Hauptreaktor, einem 0,3 L-Rührkessel als Nachreaktor und einem nachgeschalteten 0,2 L-Settler. Zum Anfahren der Versuchsanlage wurde der Hauptreaktor mit Rohaustrag, welcher gemäß Beispiel 2 gewonnen wurde und die organische und die wässrige Phase umfasste, gefüllt und auf 75°C erwärmt. Nun wurde mit der kontinuierlichen Fahrweise begonnen und bei einer Reaktionstemperatur von 75°C innerhalb von 6 Stunden 763 g (5,91 Mol) Dibutylamin, 292 g Phosgen und 1441 g 15 Gew.-%ige wässrige Natronlauge zugefahren, wobei der pH-Wert im Hauptreaktor über die Dosierung der Natronlauge auf einen Wert von etwa 11 gehalten wurde. Der Nachreaktor blieb unbeheizt. Das Reaktionsgemisch wurde kontinuierlich über einen Überlauf (Entnahme 5 cm unterhalb des Füllstandes) vom Hauptreaktor in den Nachreaktor geleitet und nach dessen Füllung in den Settler geführt. Dort erfolgte die Phasentrennung. Es wurden dabei insgesamt 815 g organische Phase erhalten. Diese enthielt 90,7 GC-Flächen-% N,N,N',N'-Tetrabutylharnstoff, 8,8 GC-Flächen-% nicht-umgesetztes Dibutylamin und 0,01 GC-Flächen-% N,N-Dibutylcarbaminsäurechlorid. Die Ausbeute an N,N,N',N'-Tetrabutylharnstoff betrug 88%, bezogen auf das eingesetzte Dibutylamin. Die während des kontinuierlichen Betriebs erreichte Raum-Zeit-Ausbeute betrug somit [(5,91 Mol/2) · 0,88 · 284 g/Mol] / 0,8 L · 6 h = 154 g/L · h.

### Beispiel 4

Die Versuchsanlage umfasste eine Rührkesselkaskade mit einem 0,5 L-Rührkessel als Hauptreaktor, einem 0,5 L-Rührkessel als Nachreaktor und einem nachgeschalteten 0,2 L-Settler. Zum Anfahren der Versuchsanlage wurde der Hauptreaktor mit Rohaustrag, welcher gemäß Beispiel 2 gewonnen wurde und die organische und die wässrige Phase umfasste, gefüllt und auf 85°C erwärmt. Nun wurde mit der kontinuierlichen Fahrweise begonnen und bei einer Reaktionstemperatur von 85°C innerhalb von 7 Stunden 1374 g (10,7 Mol) Dibutylamin, 533 g Phosgen und 2931 g 15 Gew.-%ige wässrige Natronlauge zugefahren, wobei der pH-Wert im Hauptreaktor über die Dosierung der Natronlauge auf einen Wert von etwa 11 gehalten wurde. Der Nachreaktor blieb unbeheizt. Das Reaktionsgemisch wurde kontinuierlich über einen Überlauf (Entnahme 5 cm unterhalb des Füllstandes) vom Hauptreaktor in den Nachreaktor geleitet und nach dessen Füllung in den Settler gepumpt. Dort erfolgte.die Phasentrennung. Es wurden dabei insgesamt 1522 g organische Phase erhalten. Diese enthielt 92,5 GC-Flächen-% N,N,N',N'-Tetrabutylharnstoff, 7,6 GC-Flächen-% nicht-umgesetztes Dibutylamin und 0,02 GC-Flächen-% N,N-Dibutylcarbaminsäurechlorid. Die Ausbeute an N,N,N',N'-Tetrabutylharnstoff betrug 93%, bezogen auf das eingesetzte Dibutylamin. Die während des kontinuierlichen Betriebs erreichte Raum-Zeit-Ausbeute betrug somit [(10,7 Mol/2) · 0,925 · 284 g/Mol] / 1,0 L · 7 h = 201 g/L·h.

### Beispiel 5

Die Versuchsanlage umfasste eine Rührkesselkaskade mit einem 0,3 L-Rührkessel als Hauptreaktor, einem 0,6 L-Rührkessel als Nachreaktor und einem nachgeschalteten 0,2 L-Settler. Zum Anfahren der Versuchsanlage wurde der Hauptreaktor mit Rohaustrag, welcher gemäß Beispiel 2 gewonnen wurde und die organische und die wässrige Phase umfasste, gefüllt und auf 85°C erwärmt. Nun wurde mit der kontinuierlichen Fahrweise begonnen und bei einer Reaktionstemperatur von 85°C innerhalb von 6,5 Stunden 534 g (4,14 Mol) Dibutylamin, 250 g Phosgen und 1450 g 15 Gew.-%ige wässrige Natronlauge zugefahren, wobei der pH-Wert im Hauptreaktor über die Dosierung der Natronlauge auf einen Wert von etwa 11 gehalten wurde. Der Nachreaktor blieb unbeheizt. Das Reaktionsgemisch wurde kontinuierlich über einen Überlauf vom Hauptreaktor in den Nachreaktor geleitet und nach dessen Füllung in den Settler gepumpt. Dort erfolgte die Phasentrennung. Es wurden dabei insgesamt 760 g organische Phase erhalten. Diese enthielt 91,4 GC-Flächen-% N,N,N',N'-Tetrabutylharnstoff, 8,19 GC-Flächen-% nicht-umgesetztes Dibutylamin und 0,06 GC-Flächen-% N,N-Dibutylcarbaminsäurechlorid. Die Ausbeute an N,N,N',N'-Tetrabutylharnstoff betrug 95%, bezogen auf das eingesetzte Dibutylamin. Die während des kontinuierlichen Betriebs erreichte Raum-Zeit-Ausbeute betrug somit [(4,14 Mol/2) · 0,95 · 284 g/Mol] / 0,9 L · 6,5 h = 95,5 g/L·h.

Tabelle 1 gibt eine Übersicht über die Reaktionsbedingungen und die Ergebnisse der Beispiele 1 bis 5.

Gegenüber dem bei W.A. Skinner et al. in J. Pharm. Sci. 68, 1979, Seite 391 bis 392 beschriebenen diskontinuierlichen Verfahren zur Herstellung von N,N,N',N'-Tetrabutylharnstoff, bei dem lediglich eine Raum-Zeit-Ausbeute von 14 g/L · h erreicht wurde, ermöglicht das erfindungsgemäße kontinuierliche Verfahren aus Beispiel 1 eine etwa 2,8-mal höhere Ausbeute von 39,1 g/L · h.

Ein Vergleich von Beispiel 1 mit Beispiel 3 zeigt, dass bei gleicher Reaktionstemperatur und gleichem Gesamt-Reaktionsvolumen eine kaskadierte Fahrweise mit einem Hauptreaktor und einem Nachreaktor zu einer signifikanten Erhöhung der Raum-Zeit-Ausbeute führt. Im vorliegenden Fall konnte durch die kaskadierte Fahrweise in Beispiel 3 eine knapp 4-mal höhere Raum-Zeit-Ausbeute erreicht werden als bei der einstufigen Fahrweise in Beispiel 1.

Beispiel 4 zeigt gegenüber Beispiel 3, dass durch eine Erhöhung der Reaktionstemperatur von 75°C auf 85°C und eine Vergrößerung des Nachreaktors von 0,3 L auf 0,5 L eine weitere deutliche Steigerung der Raum-Zeit-Ausbeute um etwa 25% auf 201 g/L · h möglich ist.

Beispiel 5 zeigt gegenüber Beispiel 4 den Einfluß der Größe des Hauptreaktors. Durch die Verkleinerung des Hauptreaktors von 0,5 L auf 0,3 L sank trotz Vergrößerung des Nachreaktors von 0,5 L auf 0,6 L die Raum-Zeit-Ausbeute deutlich auf etwa die Hälfte ab und betrug 95,5 g/L · h.

**Tabelle 1:**

| Übersicht über die Reaktionsbedingungen und die Ergebnisse der Beispiele 1 bis 5 | | | | | | |
|---|---|---|---|---|---|---|
| Beispiel | Anzahl der hintereinandergeschalteten Rührkessel | Volumen Hauptreaktor | Volumen Nachreaktor | Gesamtes Reaktionsvolumen | Reaktionstemperatur | Raum-Zeit-Ausbeute |
| | | [L] | [L] | [L] | [°C] | [g/L · h] |
| 1 | 1 | 0,8 | —— | 0,8 | 75 | 39,1 |
| 3 | 2 | 0,5 | 0,3 | 0,8 | 75 | 154 |
| 4 | 2 | 0,5 | 0,5 | 1,0 | 85 | 201 |
| 5 | 2 | 0,3 | 0,6 | 0,9 | 85 | 95,5 |

## Patentansprüche

1. Verfahren zur Herstellung von tetrasubstituierten Harnstoffen durch Umsetzung der entsprechenden Amine mit Phosgen in Gegenwart einer wässrigen anorganischen Base bei einer Temperatur im Bereich von 0 bis 150°C **dadurch gekennzeichnet, dass** man
das entsprechende Amin, das Phosgen und die wässrige anorganische Base dem Reaktionsapparat im Mittel kontinuierlich zuführt,
durch die Auswahl der herzustellenden tetrasubstituierten Harnstoffe, durch die Mengenverhältnisse der zuzuführenden Stoffe und Stoffgemische, durch die Temperatur während der Umsetzung und gegebenenfalls durch die Zufuhr eines organischen, mit Wasser nicht vollständig mischbaren Lösungsmittels im Reaktionsapparat ein Zweiphasensystem bildet, und
das Reaktionsgemisch aus dem Reaktionsapparat im Mittel kontinuierlich abführt.

2. Verfahren nach Anspruch 1, **dadurch gekennzeichnet, dass** die herzustellenden tetrasubstituierten Harnstoffe eine Löslichkeit in Wasser von ≤ 10 g/L Wasser, gemessen bei 25°C und Normaldruck aufweisen.

3. Verfahren nach den Ansprüchen 1 bis 2, **dadurch gekennzeichnet, dass** die herzustellenden tetrasubstituierten Harnstoffe einen Schmelzpunkt von ≤ 150°C aufweisen.

4. Verfahren nach den Ansprüchen 1 bis 3, **dadurch gekennzeichnet, dass** man als wässrige anorganische Base eine wässrige anorganische Base einsetzt, welche einen niedrigeren pK_{b}-Wert, gemessen bei 25°C in wässriger Lösung, als das entsprechende Amin aufweist.

5. Verfahren nach den Ansprüchen 1 bis 4, **dadurch gekennzeichnet, dass** man als wässrige anorganische Base wässrige Natronlauge und/oder Kalilauge einsetzt.

6. Verfahren nach den Ansprüchen 1 bis 5, **dadurch gekennzeichnet, dass** man die Umsetzung bei einem Druck im Bereich von 0,05 bis 1,0 MPa abs durchführt.

7. Verfahren nach den Ansprüchen 1 bis 6, **dadurch gekennzeichnet, dass** man die Umsetzung in einem Rührkessel durchführt.

8. Verfahren nach den Ansprüchen 1 bis 7, **dadurch gekennzeichnet, dass** man die Umsetzung in einer Kaskade von mindestens zwei Rührkesseln durchführt.

9. Verfahren nach den Ansprüchen 7 bis 8, **dadurch gekennzeichnet, dass** man einen Teil des Reaktionsgemisches aus dem flüssigkeitsoberflächennahen Bereich und einen weiteren Teil aus dem bodennahen Bereich des Rührkessels abführt.

10. Verfahren nach den Ansprüchen 1 bis 9, **dadurch gekennzeichnet, dass** man als tetrasubstituierte Harnstoffe N,N,N',N'-Tetrabutylharnstoff, N,N'-Dimethylethylenharnstoff und/oder N,N'-Dimethypropylenharnstoff herstellt.

## Claims

1. A process for the preparation of tetrasubstituted ureas by reaction of the corresponding amines with phosgene in the presence of an aqueous inorganic base at a temperature in the range from 0 to 150°C, which comprises
feeding the corresponding amine, the phosgene and the aqueous inorganic base on average continuously to the reaction apparatus,
forming a two-phase system in the reaction apparatus through the choice of the tetrasubstituted ureas to be prepared, through the mixing ratios of the substances and substance mixtures to be fed in, through the temperature during the reaction and, where appropriate, through the feed of an organic solvent which is not completely miscible with water, and
discharging the reaction mixture on average continuously from the reaction apparatus.

2. The process according to claim 1, wherein the tetrasubstituted ureas to be prepared have a solubility in water of ≤ 10 g/l of water, measured at 25°C and atmospheric pressure.

3. The process according to either of claims 1 and 2, wherein the tetrasubstituted ureas to be prepared have a melting point of ≤ 150°C.

4. The process according to any one of claims 1 to 3, wherein the aqueous inorganic base employed is an aqueous inorganic base which has a lower pK_{b} value, measured at 25°C in aqueous solution, than the corresponding amine.

5. The process according to any one of claims 1 to 4, wherein the aqueous inorganic base employed is aqueous sodium hydroxide solution and/or potassium hydroxide solution.

6. The process according to any one of claims 1 to 5, wherein the reaction is carried out at a pressure in the range from 0.05 to 1.0 MPabs.

7. The process according to any one of claims 1 to 6, wherein the reaction is carried out in a stirred-tank reactor.

8. The process according to any one of claims 1 to 7, wherein the reaction is carried out in a cascade of at least two stirred-tank reactors.

9. The process according to either of claims 7 and 8, wherein part of the reaction mixture is discharged from the region close to the liquid surface and a further part is discharged from the region close to the bottom of the stirred-tank reactor.

10. The process according to any one of claims 1 to 9, wherein the tetrasubstituted ureas prepared are N,N,N',N'-tetrabutylurea, N,N'-dimethylethyleneurea and/or N,N'-dimethylpropyleneurea.

## Revendications

1. Procédé pour la production d'urées tétrasubstituées, par mise en réaction des amines correspondantes avec du phosgène en présence d'une base inorganique aqueuse, à une température dans la plage de 0 à 150 °C, **caractérisé en ce que**
on introduit en moyenne en continu dans l'appareil de réaction l'amine correspondante, le phosgène et la base inorganique aqueuse,
par le choix des urées tétrasubstituées à produire, par les proportions des substances et mélanges de substances à ajouter, par la température pendant la réaction et éventuellement par l'addition d'un solvant organique, non totalement miscible à l'eau, il se forme un système biphasique dans l'appareil de réaction, et
on évacue en moyenne en continu le mélange réactionnel de l'appareil de réaction.

2. Procédé selon la revendication 1, **caractérisé en ce que** les urées tétrasubstituées à produire présentent une solubilité dans l'eau de ≤ 10 g/l d'eau, mesurée à 25 °C et sous la pression normale.

3. Procédé selon les revendications 1 et 2, **caractérisé en ce que** les urées tétrasubstituées à produire présentent un point de fusion de ≤ 150 °C.

4. Procédé selon les revendications 1 à 3, **caractérisé en ce qu'**on utilise comme base inorganique aqueuse une base inorganique aqueuse qui présente un indice pK_{b}, mesuré à 25 °C en solution aqueuse, inférieur à celui de l'amine correspondante.

5. Procédé selon les revendications 1 à 4, **caractérisé en ce qu'**on utilise comme base inorganique aqueuse une solution d'hydroxyde de sodium et/ou une solution d'hydroxyde de potassium.

6. Procédé selon les revendications 1 à 5, **caractérisé en ce qu'**on effectue la réaction sous une pression dans la plage de 0,05 à 1,0 MPa (absolue).

7. Procédé selon les revendications 1 à 6, **caractérisé en ce qu'**on effectue la réaction dans un récipient à agitation.

8. Procédé selon les revendications 1 à 7, **caractérisé en ce que** ce qu'on effectue la réaction dans une cascade d'au moins deux récipients à agitation.

9. Procédé selon les revendications 7 et 8, **caractérisé en ce que** ce qu'on évacue une partie du mélange réactionnel à partir de la zone voisine de la surface du liquide et une autre partie à partir de la zone voisine du fond du récipient à agitation.

10. Procédé selon les revendications 1 à 9, **caractérisé en ce que** comme urées tétrasubstituées on produit de la N,N,N',N'-tétrabutylurée, de la N,N'-diméthyléthylène-urée et/ou de la N,N'-diméthylpropylène-urée.
